# EUROPEAN PATENT SPECIFICATION

(11) **EP 2 157 140 B1**
(45) Date of publication and mention of the grant of the patent: **14.03.2018**
(21) Application number: 08740260.8
(22) Date of filing: 11.04.2008
(51) Int. Cl.: A61K 8/02, A61K 8/11, A61K 8/26, A61K 8/891, C03B 37/005, C03C 17/30, C09C 1/00, A61Q 1/02

(54) **FLAKY GLASS AND COSMETIC PREPARATION WITH FLAKY GLASS MIXED THEREIN**
PLÄTTCHENFÖRMIGES GLAS SOWIE KOSMETIKUM MIT DARIN EINGEMISCHTEM PLÄTTCHENFÖRMIGEN GLAS
VERRE EN PAILLETTES ET PRÉPARATION COSMÉTIQUE DANS LAQUELLE EST MÉLANGÉ DU VERRE EN PAILLETTES

(30) Priority: 25.04.2007 JP 2007116175
(43) Date of publication of application: 24.02.2010
(73) Proprietor: Nippon Sheet Glass Company, Limited, Tokyo 108-6321 (JP)
(72) Inventor: MAEDA, Takeshi, Tokyo 108-6321 (JP); YAGYU, Tomohiro, Tokyo 108-6321 (JP); OTANI, Kazuhiro, Tokyo 108-6321 (JP)
(74) Representative: Hart-Davis, Jason
(86) International application number: PCT/JP2008/057163
(87) International publication number: WO 2008/133042

(56) References cited:
- EP-A1- 1 593 366
- WO-A1-2006/068255
- JP-A- 03 243 667
- JP-A- 62 045 666
- JP-A- 2001 262 004
- JP-A- 2002 038 051
- JP-A- 2006 257 176
- JP-A- 2007 176 937

## Description

### TECHNICAL FIELD

The present invention relates to glass flakes and a cosmetic containing the glass flakes.

### BACKGROUND ART

In makeup cosmetics such as face powder, powder foundation, cheek rouge and eye shadow, natural flaky extender pigments such as talc, mica and sericite are mainly used. In particular, mica, sericite and silica flakes (flaky silica) are transparent and soft to the touch, and are removed easily when they are processed into pressed cake type cosmetics. Thus, they are used widely.

However, since mica and sericite are natural products, they contain impurities such as iron. Because of this, they tend to darken when oil is added thereto, which causes a problem in that good color development cannot be obtained. Furthermore, mica and sericite do not show sufficient adhesion to the skin and smoothness.

Meanwhile, synthetic mica and amorphous flaky silica (see, for example, JP 6(1994)-87720 A), and crystalline flaky silica (see, for example, JP 4(1992)-145011 A) also are used instead of the natural products. However, these materials suffer from such a problem that they do not have sufficient strength and thus are crushed when they are mixed with other powder materials. The above-mentioned amorphous flaky silica and crystalline flaky silica are obtained by applying a silica coating on a substrate such as a stainless steel sheet and a steel sheet, drying it, and then peeling off the coating layer from the substrate. Therefore, asperities on the surface of the substrate are transferred to flaky silica, resulting in a product with the asperities being formed on one surface thereof (the surface that has come into contact with the substrate during production thereof), which causes surface reflection. As a result, it is difficult to maintain the transparency of the silica flakes, and the obtained silica flakes are too glossy.

Furthermore, makeup cosmetics containing irregular-shaped silica gel also are proposed. However, since silica gel is a porous material, cosmetics containing such silica gel tend to absorb moisture of the skin, resulting in rough feeling and poor spreading on the skin.

On the other hand, mica and synthetic mica are too glossy. Thus, cosmetics containing these also have a problem that a natural makeup effect cannot be obtained due to their excessive gloss, that is, a natural skin appearance hardly can be obtained.

Further, even though there also are proposed cosmetics that have improved smoothness on the skin or improved adhesion to the skin due to a surface treatment to extender pigments (by coating the surface of the extender pigments with a particular treatment agent), sufficient smoothness has not been obtained.

According to EP 1 593 366 A1, a pearlescent pigment that is a coloring agent containing a thin base material and a coating layer, wherein the base material is selected from the group consisting of mica, synthetic mica, silica and glass, and the coating layer is selected from the group consisting of metal, metal oxide, metallic complex, and organic pigment, is subjected to surface treatment like a silicone treatment, a fatty acid treatment, an amino acid treatment, a lecithin treatment, a metal soap treatment, an alkyl treatment, a fluorine compound treatment, an ester treatment and a combined use of these treatments, and used for a cosmetic powder.

WO 2006/068255 A1 teaches a scaly glass with a coating film comprising a metal and/or a metal oxide as a main component and covering the surface of the scaly grass. US2005049133 discloses the preparation of glass flakes having an average thickness t of 0.1-100 µm and an aspect ratio (average particle diameter a/average thickness t) of 2-1000, optionally coated with a silicone compound, which can be used in cosmetics.

### DISCLOSURE OF THE INVENTION

It is an object of the present invention to solve the above-mentioned problems and to provide glass flakes that are used to achieve cosmetics that are smooth and comfortable to the touch, are excellent in transparency and adhesion to the skin, and have a natural finish with moderate gloss, that is, a so-called natural skin appearance. Further, it is another object of the present invention to provide a cosmetic containing such glass flakes.

The glass flakes of the present invention are defined in claim 1.

It should be noted that the average thickness of the glass flake substrates herein means a numerical value obtained by, after extracting at least 100 pieces of the glass flake substrates, measuring the thickness of each glass flake substrate with a scanning electron microscope (SEM), and then dividing the total of the thickness by the number of the measured pieces. Further, the average particle size of the glass flake substrates herein means a particle size corresponding to the cumulative mass percent of 50% (D50) in a particle size distribution measured based on a laser diffraction/scattering method.

The cosmetic of the present invention contains the glass flakes of the present invention described above. layer

The glass flakes of the present invention include the coating layer formed of methyl hydrogen silicone at a content percentage of 0.05 to 2.50 mass%, as determined by ignition loss at 625 ± 20°C. Because of this, the cosmetic containing the glass flakes of the present invention is smooth and comfortable to the touch, is excellent in transparency, color development property and adhesion to the skin, and enables a natural finish with moderate gloss, that is, a makeup with a so-called natural skin appearance.

### BEST MODE FOR CARRYING OUT THE INVENTION

Hereinafter, embodiments of the glass flakes and cosmetic of the present invention are described.

The glass flakes of the present embodiment each are provided with a coating layer formed of methyl hydrogen silicone on the surface of a glass flake substrate. The content percentage of the coating layer in the glass flakes of the present embodiment is in the range from 0.05 to 2.50 mass% as determined by ignition loss at 625 ± 20°C. In other words, the glass flakes of the present embodiment are surface-treated with 0.05 to 2.50 mass% of methyl hydrogen silicone as determined by ignition loss. In the glass flakes of the present embodiment, the surface of the glass flake substrates may include a part that has not been coated by the coating layer. layed

More preferably, the content percentage of the coating layer formed of methyl hydrogen silicone is in the range from 0.10 to 1.00 mass% as determined by ignition loss.

If the coating amount of methyl hydrogen silicone in the glass flakes (the content percentage of the coating layer) is less than 0.05 mass% as determined by ignition loss from the glass flakes, the water-repellent effect of the glass flakes is insufficient. For this reason, when contained in cosmetics, such glass flakes absorb sweat on the skin, which is likely to cause a dull appearance. On the other hand, if the coating amount is more than 2.50 mass% as determined by ignition loss from the glass flakes, the glass flakes tend to agglomerate, resulting in a problem that they are difficult to disperse uniformly in cosmetics. Accordingly, in the present embodiment, the coating amount of methyl hydrogen silicone in the glass flakes is to be within the above range.

In the glass flakes of the present invention, since the content percentage of the coating layers is determined by ignition loss, the range in which advantageous effects are achieved can be specified more precisely, compared to the case in which the content percentage of the coating layer is determined by design. Thus, according to the glass flakes of the present invention, it is possible to provide a cosmetic that is smooth and comfortable to the touch, is excellent in transparency and adhesion to the skin, and has a natural finish with moderate gloss, that is, a so-called natural skin appearance, more surely and steadily.

Examples of methyl hydrogen silicone to be used in the present embodiment include "KF-99" and "KF-9901" manufactured by Shin-Etsu Chemical Co., Ltd., and "SH1107" and "BY16-805" manufactured by Dow Corning Toray Co., Ltd.. As a method for coating the glass flake substrates with methyl hydrogen silicone, a solution immersion method, a solution spray method or the like may be employed, as exemplified in JP 61(1986)-73775 A, JP 61(1986)-17667 A, and JP 63(1988)-201041 A. For example, coating layers made of methyl hydrogen silicone may be formed by diluting methyl hydrogen silicone with aromatic hydrocarbons such as toluene and xylene, aliphatic hydrocarbons such as petroleum ether, petroleum spirit and kerosene, or isopropyl alcohol, and applying it to glass flake substrates followed by heat drying of the coating layers. In this case, the amount of methyl hydrogen silicone should be adjusted appropriately so that the content percentage of the coating layer to be formed is within the range from 0.10 to 2.50 mass% as determined by ignition loss (preferably from 0.10 to 1.00 mass%), and then the methyl hydrogen silicone is applied to the glass flake substrates.

The glass flake substrates to be used in the present embodiment can be prepared by, for example, a so-called blowing method as disclosed in JP 45(1970)-3541 B, and a so-called rotary method as disclosed in JP 59(1984)-21533 A and JP 2(1990)-503669 T.

In the blowing method, a nozzle is introduced into a liquid tank containing molten glass, air is blown through the nozzle to inflate the molten glass into a so-called balloon shape, and it is drawn out by rollers. Thus, glass flakes are obtained. In the rotary method, molten glass is poured continuously into a rapidly rotating flat plate or bowl, and the molten glass is stretched over the rim of the plate or the bowl. Thus, glass flakes are obtained.

According to these methods, glass flake substrates having an average thickness of 0.1 to 5.0 µm, and an average particle size of 1 to 1000 µm can be obtained. However, glass flake substrates to be used in the present embodiment have an average thickness of 0.1 to 1.0 µm, and an average particle size of 1 to 100 µm because, when contained in cosmetics, such glass flake substrates can achieve high transparency and adhesion to the skin, and a natural finish with moderate gloss (natural skin appearance).

Although examples of the glass flake substrates to be used in the present embodiment include glass compositions that are generally referred to as E-glass and C-glass, elution of boric oxide resulting from the composition is a concern. Recently, there is a growing demand for safety of cosmetics, and the "Japanese Cosmetic Ingredients Codex" supervised by Pharmaceutical Affairs Bureau, Examination Division of Ministry of Health and Welfare (issued by YAKUJI NIPPO LIMITED. in 1997) prescribes in the category of "boron nitride" that the elution amount of boron should be 20 ppm or less. Accordingly, it is desirable that the glass to be used for the glass flakes that are contained in cosmetics should be free from boron as a component. Examples of such glass include boron-free E-glass as disclosed in JP 2002-226732 A.

The glass flakes described in the present embodiment each include a glass flake substrate and a coating layer formed of methyl hydrogen silicone that is provided on the surface of the glass flake substrate.

The cosmetic of the present embodiment contains the above-described glass flakes of the present embodiment.

In addition to the above-mentioned glass flakes, other components that are used commonly for cosmetics can be mixed suitably in the cosmetic of the present embodiment as required. Examples of such other components include inorganic powders, organic powders, pigments, dyes, oil components, organic solvents, resins, and plasticizers. Examples of the inorganic powders include talc, kaolin, mica, sericite, other types of micas, magnesium carbonate, calcium carbonate, aluminum silicate, magnesium silicate, and clays.

Examples of the organic powders include powders such as nylon powder, polyethylene powder, polystyrene powder, epoxy powder, acrylic powder, and plastic beads made of nylon, acrylic, or the like.

Examples of the pigments include: inorganic white pigments such as microcrystalline cellulose, titanium oxide, zinc oxide, and zirconium oxide; inorganic red pigments such as iron oxide, and iron titanate; inorganic yellow pigments such as yellow iron oxide, and yellow ocher; inorganic black pigments such as black iron oxide, and carbon black; inorganic green pigments such as chromium oxide, chromium hydroxide, and cobalt titanate; inorganic blue pigments such as iron blue; pearl luster pigments such as glass flakes and silica coated with titanium oxide or iron oxide, titanium oxide coated mica (mica powder coated with titanium oxide), and bismuth oxychloride; and metallic powder pigments such as aluminum powder and copper powder.

Examples of the dyes include: organic pigments such as red color No. 201, red color No. 202, red color No. 204, red color No. 205, red color No. 220, red color No. 226, red color No. 228, red color No. 405, orange color No. 203, orange color No. 204, yellow color No. 205, yellow color No. 401, and blue color No. 404; organic pigments such as zirconium lake, barium lake, and aluminum lake of red color No. 3, red color No. 104, red color No. 106, red color No. 227, red color No. 230, red color No. 401, red color No. 505, orange color No. 205, yellow color No. 4, yellow color No. 5, yellow color No. 202, yellow color No. 203, green color No. 3, and blue color No. 1; and natural dyes such as chlorophyll and beta-carotene.

Examples of the oil components include hydrocarbons such as squalane, liquid paraffin, Vaseline, microcrystalline wax, ozokerite, ceresin, myristic acid, palmitic acid, stearic acid, oleic acid, isostearic acid, cetyl alcohol, hexadecyl alcohol, oleylalcohol, cetyl-2-ethylhexanoate, 2-ethylhexyl palmitate, 2-octyldodecyl myristate, neopentyl glycol di-2-ethylhexanoate, glycerol tri-2-ethylhexanoate, 2-octyldodecyl oleate, isopropyl myristate, glycerol triisostearate, glycerol tri-coconut oil fatty acid, olive oil, avocado oil, yellow beeswax, myristyl myristate, mink oil, and lanoline; silicone oil; higher fatty acid; esters of oils and fats; higher alcohol; and wax.

Further, organic solvents such as acetone, toluene, butyl acetate, and acetate ester; resins such as alkyd resin and urea resin; plasticizers such as camphor and acetyl tributyl citrate; ultraviolet absorbers; antioxidants; preservatives; surfactants; moisturizers; fragrances; water; alcohol; and thickeners also may be contained therein.

There are various forms of cosmetics. Examples thereof include powder, cake, pencil, stick, ointment, liquid, emulsion, and cream forms. Examples of these cosmetics include facial skin care cosmetics such as skin lotion, skin milk, and skin cream; and makeup cosmetics such as foundation, lipstick, eye shadow, cheek rouge, eyeliner, nail enamel, and mascara.

### EXAMPLES

Hereinafter, the present invention is described by way of examples and comparative examples in more detail, but the present invention is not limited to the following examples within the scope of the present invention.

### (Examples 1 to 12)

Samples of the glass flakes of the present invention were prepared as Examples 1 to 12.

Glass flake substrates were prepared by a blowing method, using E-glass and glass not containing boron (boron-free glass) that each have the composition indicated in Table 1. Specifically, glass with the E-glass composition or boron-free composition was put into a melting tank that had been heated to 1200°C or higher to be melted. Next, a nozzle was introduced into the melting tank, the molten glass was inflated with air fed through the nozzle to be formed into thin glass, and the thin glass was continuously drawn out of the tank by rollers. Glass with an average thickness of 0.4 µm and glass with an average thickness of 0.7 µm were obtained by changing the amount of air to be fed and the rotational speed of the rollers. After that, the glass was pulverized and classified, and thus glass flake substrates with an average particle size of 10 µm and glass flake substrates with an average particle size of 25 µm were obtained from each. The pulverization was carried out with a jet mill type pulverizer (product name "Labojet LJ" manufactured by Nippon Pneumatic Mfg. Co., Ltd.). The particle size was adjusted, using sieve classification. The sieve classification was carried out with an electromagnetic sieve shaker (product name "RETSCH SIEVE SHAKER, type VIBRO" manufactured by RETSCH Co., Ltd.), and a sieve was selected appropriately depending on the target particle size distribution. The average particle size was determined with a laser diffraction particle size distribution analyzer (product name "Microtrack (TM) HRA" manufactured by Nikkiso Co., Ltd.).

**Table 1**

| Component | E-glass | Boron-free glass |
|---|---|---|
| SiO₂ | 54.7 | 61.1 |
| Al₂O₃ | 14.0 | 11.2 |
| CaO | 23.4 | 21.7 |
| MgO | 0.3 | 3.1 |
| Na₂O | 0.4 | 1.3 |
| K₂O | 0.2 | 0.6 |
| Li₂O | - | 0.6 |
| B₂O₃ | 5.8 | - |
| Others | 1.2 | 0.4 |

A coating layer made of methyl hydrogen silicone ("KF-9901" manufactured by Shin-Etsu Chemical Co., Ltd.) was formed on each of the obtained glass flake substrates by the following method. First, methyl hydrogen silicone was diluted with isopropyl alcohol. Then, application of it to the glass flake substrates was carried out by a solution immersion method, followed by drying of the coating layers at 70°C for 4 hours and baking at 160°C for 2 hours, so that the coating layers were formed. Thus, samples of glass flakes of Examples 1 to 12 were prepared. Table 2 indicates the type of glass used for the glass flakes, and the average thickness and average particle size of the glass flake substrates for each Example 1 to 12.

Subsequently, the method for determining the ignition loss for each sample will be described. The above-prepared glass flakes of each Example 1 to 12 (the glass flakes in which the surface of each glass flake substrate was provided with a coating layer made of methyl hydrogen silicone) were dried at 110 ± 5°C for at least 60 minutes, and then were subjected to ignition at 625°C for at least 15 minutes. After that, the mass reduction (loss) of the glass flakes due to this ignition was determined so that the percentage of the loss from the mass of the glass flakes before ignition was calculated. Thus the ignition loss was obtained. Table 2 indicates the ignition loss of the glass flakes for each Example 1 to 12.

**Table 2**

| | EX. 1 | EX. 2 | EX. 3 | EX. 4 |
|---|---|---|---|---|
| Glass Type | E-Glass | E-Glass | E-Glass | E-Glass |
| Average Thickness (µm) | 0.4 | 0.4 | 0.4 | 0.7 |
| Average Particle Size (µm) | 10 | 10 | 25 | 10 |
| Ignition Loss (mass%) | 0.05 | 0.08 | 0.95 | 0.15 |
| Material of Coating Layer | Methyl Hydrogen Silicone | | | |

| | EX. 5 | EX. 6 | EX. 7 | EX. 8 |
|---|---|---|---|---|
| Glass Type | E-Glass | E-Glass | Boron-free Glass | Boron-free Glass |
| Average Thickness (µm) | 0.7 | 0.7 | 0.4 | 0.4 |
| Average Particle Size (µm) | 25 | 25 | 10 | 10 |
| Ignition Loss (mass%) | 2.39 | 2.45 | 0.10 | 0.26 |
| Material of Coating Layer | Methyl Hydrogen Silicone | | | |

| | EX. 9 | EX. 10 | EX. 11 | EX. 12 |
|---|---|---|---|---|
| Glass Type | Boron-free Glass | Boron-free Glass | Boron-free Glass | Boron-free Glass |
| Average Thickness (µm) | 0.4 | 0.7 | 0.7 | 0.7 |
| Average Particle Size (µm) | 25 | 10 | 25 | 25 |
| Ignition Loss (mass%) | 2.32 | 0.11 | 1.33 | 2.27 |
| Material of Coating Layer | Methyl Hydrogen Silicone | | | |

### (Comparative examples 1 to 12)

As Comparative examples, glass flakes without methyl hydrogen silicone coating glass flake substrates (Comparative examples 1 and 2), glass flakes with less than 0.05 mass% of methyl hydrogen silicone as determined by ignition loss coating glass flake substrates (Comparative example 3,4 and 6), and glass flakes with more than 2.50 mass% of methyl hydrogen silicone as determined by ignition loss coating glass flake substrates (Comparative examples 5, 7 and 8) were prepared. In this regard, each of the glass flake substrates were prepared in the same manner as Examples 1 to 12.

As additional Comparative examples, glass flakes with methyltrimethoxysilane that serves as a water-repellent silane coupling agent coating glass flake substrates that had been prepared in the same manner as Examples 1 to 12 (Comparative examples 9 and 10) were prepared.

As further additional Comparative examples, using natural mica and synthetic mica instead of the glass flake substrates, samples with methyl hydrogen silicone coating the surface of the natural mica and synthetic mica in the same manner as Examples 1 to 12 (Comparative examples 11 and 12) were prepared.

Table 3 indicates the type (the type of glass or the type of mica) of the substrates used in each Comparative example 1 to 12, the average thickness and average particle size thereof, the ignition loss that was determined in the same manner as Examples 1 to 12, and the material of the coating layers.

**Table 3**

| | C.EX. 1 | C.EX. 2 | | C.EX. 3 | C.EX. 4 |
|---|---|---|---|---|---|
| Glass Type | E-Glass | Boron-free Glass | | E-Glass | E-Glass |
| Average Thickness (µm) | 0.4 | 0.4 | | 0.4 | 0.7 |
| Average Particle Size (µm) | 10 | 10 | | 10 | 25 |
| Ignition Loss (mass%) | - | - | | 0.03 | 0.04 |
| Material of Coating Layer | No Coating Layer | | | Methyl Hydrogen Silicone | |

| | C.EX. 5 | C.EX. 6 | | C.EX. 7 | C.EX. 8 |
|---|---|---|---|---|---|
| Glass Type or Mica Type | E-Glass | Boron-free Glass | | Boron-free Glass | Boron-free Glass |
| Average Thickness (µm) | 0.7 | 0.4 | | 0.4 | 0.7 |
| Average Particle Size (µm) | 25 | 10 | | 10 | 25 |
| Ignition Loss (mass%) | 2.64 | 0.04 | | 2.58 | 2.89 |
| Material of Coating Layer | Methyl Hydrogen Silicone | | | | |

| | C.EX. 9 | | C.EX. 10 | C.EX. 11 | C.EX. 12 |
|---|---|---|---|---|---|
| Glass Type or Mica Type | Boron-free Glass | | Boron-free Glass | Natural Mica | Synthetic Mica |
| Average Thickness (µm) | 0.4 | | 0.4 | - | - |
| Average Particle Size (µm) | 10 | | 10 | 10 | 10 |
| Ignition Loss (mass%) | 0.30 | | 0.76 | 0.57 | 0.44 |
| Material of Coating Layer | Methyltrimethoxysilane | | | Methyl Hydrogen Silicone | |

### (Examples 13 to 24 and Comparative Examples 13 to 24)

Powder foundations containing components indicated in Table 4 were prepared, using the glass flakes of Examples 1 to 12 and the samples of Comparative Examples 1 to 12, and evaluations as cosmetics were made Examples 13 to 24 and Comparative Examples 13 to 24.

The components (1) to (8) indicated in Table 4 were mixed by a Henschel mixer. The components (9) to (13) dissolved and mixed by heat were added to this mixture, and then it was pulverized by a pulverizer. The resultant powder was molded into a plate of 6 cm in diameter at a pressure of 1.5 kg/cm². Thus, each powder foundation was obtained.

**Table 4**

| | Component | Part(s) by Mass |
|---|---|---|
| (1) | Glass Flakes of EX. 1 to 12 or Samples of C. EX. 1 to 12 | 35 parts by mass |
| (2) | Talc | 20 parts by mass |
| (3) | Glass Flakes with Titanium Coating Layer | 20 parts by mass |
| (4) | Titanium dioxide | 10 parts by mass |
| (5) | Spherical Polyethylene Powder | 5 parts by mass |
| (6) | Colcothar | 1.0 part by mass |
| (7) | Yellow Iron Oxide | 3.0 parts by mass |
| (8) | Black Iron Oxide | 0.1 part by mass |
| (9) | Silicone Oil | 1 part by mass |
| (10) | 2-Ethylhexyl Palmitate | 9 parts by mass |
| (11) | Sorbitan Sesquioleate | 1 part by mass |
| (12) | Preservative | 0.3 part by mass |
| (13) | Fragrance | 0.1 part by mass |

For Examples 13 to 24 and Comparative Examples 13 to 24 in which powder foundations were prepared using glass flakes of Examples 1 to 12 and samples of Comparative Examples 1 to 12, a sensory test was conducted.

For the sensory test, 10 panelists were employed. The average of the evaluations by the 10 panelists was employed as the evaluation of feeling in use of the cosmetics. Table 5 indicates the evaluation criteria, and Table 6 indicates the evaluation results.

**Table 5**

| Evaluation Score | Feeling of Finish | Feeling of Transparency | Feeling of Use (Spreading) |
|---|---|---|---|
| 1 | Very Unnatural | Very Poor | Very Poor |
| 2 | Unnatural | Poor | Poor |
| 3 | Average | Average | Average |
| 4 | Natural | Good | Good |
| 5 | Very Natural | Very Good | Very Good |

**Table 6**

| | EX. 13 | EX. 14 | EX. 15 | EX. 16 |
|---|---|---|---|---|
| Substrate Used | EX. 1 | EX. 2 | EX. 3 | EX. 4 |
| Elution Amount of Boron (ppm) | 10 | 6 | n.d.(*1) | 1 |
| Feeling of Finish | 5 | 5 | 5 | 5 |
| Feeling of Transparency | 5 | 5 | 5 | 5 |
| Feeling of Use (Spreading) | 4.5 | 4.6 | 4.8 | 4.6 |

| | EX. 17 | EX. 18 | EX. 19 | EX. 20 |
|---|---|---|---|---|
| Substrate Used | EX. 5 | EX. 6 | EX. 7 | EX. 8 |
| Elution Amount of Boron (ppm) | n.d.(*1) | n.d.(*1) | n.d.(*1) | n.d.(*1) |
| Feeling of Finish | 4.9 | 4.8 | 5 | 5 |
| Feeling of Transparency | 4.5 | 4.5 | 5 | 5 |
| Feeling of Use (Spreading) | 4.7 | 4.8 | 4.6 | 5 |

| | EX. 21 | EX. 22 | EX. 23 | EX. 24 |
|---|---|---|---|---|
| Substrate Used | EX. 9 | EX. 10 | EX. 11 | EX. 12 |
| Elution Amount of Boron (ppm) | n.d.(*1) | n.d.(*1) | n.d.(*1) | n.d.(*1) |
| Feeling of Finish | 5 | 5 | 4.9 | 4.9 |
| Feeling of Transparency | 4.8 | 5 | 4.7 | 4.6 |
| Feeling of Use (Spreading) | 4.7 | 4.7 | 5 | 4.7 |

| | C.EX. 13 | C.EX. 14 | C.EX. 15 | C.EX. 16 |
|---|---|---|---|---|
| Substrate Used | C.EX. 1 | C.EX. 2 | C.EX. 3 | C.EX. 4 |
| Elution Amount of Boron (ppm) | 52 | n.d.(*1) | 19 | 13 |
| Feeling of Finish | 3.7 | 3.8 | 3.8 | 3.9 |
| Feeling of Transparency | 4.3 | 4.2 | 4.4 | 4.4 |
| Feeling of Use (Spreading) | 3.6 | 3.8 | 3.9 | 4.0 |

| | C.EX. 17 | C.EX. 18 | C.EX. 19 | C.EX. 20 |
|---|---|---|---|---|
| Substrate Used | C.EX. 5 | C.EX. 6 | C.EX. 7 | C.EX. 8 |
| Elution Amount of Boron (ppm) | n.d.(*1) | n.d.(*1) | n.d.(*1) | n.d.(*1) |
| Feeling of Finish | 4.7 | 3.9 | 4.7 | 4.8 |
| Feeling of Transparency | 3.9 | 4.4 | 3.9 | 3.9 |
| Feeling of Use (Spreading) | 4.1 | 4.2 | 4.2 | 4.1 |

| | C.EX. 21 | C.EX. 22 | C.EX. 23 | C.EX. 24 |
|---|---|---|---|---|
| Substrate Used | C.EX. 9 | C.EX. 10 | C.EX. 11 | C.EX. 12 |
| Elution Amount of Boron (ppm) | n.d.(*1) | n.d.(*1) | - | - |
| Feeling of Finish | 3.5 | 3.2 | 3.9 | 4.1 |
| Feeling of Transparency | 3.7 | 3.4 | 2.8 | 3.7 |
| Feeling of Use (Spreading) | 3.3 | 3.1 | 4.8 | 4.9 |

| | | | | |
|---|---|---|---|---|
| (*1) n.d.: below 0.25 ppm that is the lower limit of quantitation | | | | |

As indicated in Table 6, the powder foundations (Examples 13 to 24) using the glass flakes of the present invention (Examples 1 to 12) satisfied an evaluation score of 4.5 or more in all the evaluation items, and thus obtained good results, compared to the powder foundations (Comparative examples 13 to 24) using the samples of Comparative examples 1 to 12.

The glass flakes of Comparative examples 1 and 2 without coating layers formed of methyl hydrogen silicone themselves were not smooth enough, resulting in poor spreading of the foundation on the skin and poor feeling of finish (Comparative examples 13 and 14). The glass flakes of Comparative examples 3, 4 and 6 with a small coating amount of methyl hydrogen silicone did not have sufficient water repellency, resulting in poor spreading and feeling of finish (Comparative examples 15, 16 and 18). The glass flakes of Comparative examples 5, 7 and 8 with a large coating amount of methyl hydrogen silicone were not able to disperse sufficiently in the foundation, and thus darkness was likely to occur in the foundation, in addition to poor feeling of use (Comparative examples 17, 19 and 20). The glass flakes of Comparative examples 9 and 10 with coating layers formed of a water-repellent silane coupling agent themselves were likely to agglomerate and did not disperse sufficiently in the foundation, and thus darkness was likely to occur in the foundation, in addition to poor feeling of finish and feeling of use (Comparative examples 21 and 22). The samples of Comparative examples 11 and 12 in which the natural mica and synthetic mica were coated by methyl hydrogen silicone did not have sufficient transparency, resulting in poor feeling of natural finish, although they had good spreading of the foundation (Comparative examples 23 and 24).

Moreover, the elution amount of boron from the glass flakes in Examples was very small, which indicates a higher safety profile, compared to Comparative examples in which E-glass was used for glass flake substrates. It should be noted that the elution amount of boron was determined by preparing a test solution in compliance with the method of "boron nitride" purity test (4), the elution amount of boron, in the Japanese Cosmetic Ingredients Codex, and quantitatively analyzing it by the atomic absorption method. The method for preparing the test solution is as follows.

2.5 g of glass flakes is put into a beaker made of fluororesin, and then 10 mL of ethanol is added thereto and stirred well. After 40 mL of water is added thereto and stirred well, it is placed on a watch glass made of fluororesin, and heated on a hot plate for 1 hour. After cooling, it is filtered, and the residues are washed with a small amount of water. Then, the wash liquid is mixed to the filtrate. This mixed liquid further is filtered with a membrane filter (0.22 µm). All the filtrate is poured into a beaker made of fluororesin, and 1 mL of sulfuric acid is added thereto. Then it is boiled on a hot plate for 10 minutes. After cooling, thus obtained solution is poured into a volumetric flask made of polyethylene. The beaker made of fluororesin is washed with a small amount of water, and the wash liquid is mixed to the solution in the volumetric flask made of polyethylene. Thereafter, water is added thereto until the amount is precisely 50 mL, and thus a test solution is obtained.

### INDUSTRIAL APPLICABILITY

The cosmetics using the glass flakes of the present invention are smooth and comfortable to the touch, are excellent in transparency and adhesion to the skin, and have a natural finish with moderate gloss, that is, a so-called natural skin appearance. Thus, the glass flakes of the present invention are useful for cosmetics.

## Claims

1. Glass flakes each consisting of
a glass flake substrate and
a coating layer coating the surface of the glass flake substrate, wherein:
the glass flake substrates have an average thickness of 0.1 to 1.0 µm, and an average particle size of 1 to 100 µm;
the coating layer is formed of methyl hydrogen silicone; and
the content percentage of the coating layer is 0.05 to 2.50 mass% as determined by subjecting said glass flakes to drying at 110 ± 5° C for at least 60 minutes followed by ignition at 625 ± 20° C for at least 15 minutes, and then determining the mass reduction of the glass flakes due to this ignition so that the percentage of the loss from the mass of the glass flakes before ignition is calculated.

2. The glass flakes according to claim 1, wherein said content percentage of the coating layer is 0.10 to 1.00 mass%.

3. A cosmetic containing the glass flakes according to claim 1.

## Patentansprüche

1. Glasflocken, jeweils bestehend aus
einem Glasflockensubstrat und
einer Beschichtungsschicht, die die Oberfläche des Glasflockensubstrates bedeckt, wobei
das Glasflockensubstrat eine durchschnittliche Dicke von 0,1 bis 1,0 µm und eine durchschnittliche Partikelgröße von 1 bis 100 µm aufweist,
die Beschichtungsschicht aus Methylhydrogensilikon gebildet ist und
der prozentuale Gehalt der Beschichtungsschicht 0,05 bis 2,50 Massen-% beträgt, der durch Unterziehen der Glasflocken einem Trocknen bei 100 ± 5 °C für wenigstens 60 Minuten, gefolgt von einem Brennen bei 625 ± 20 °C für wenigstens 15 Minuten und dann Bestimmen der Massenreduzierung der Glasflocken aufgrund dieses Brennens bestimmt wird, so dass der prozentuale Massenverlust gegenüber den Glasflocken vor dem Brennen berechnet wird.

2. Glasflocken nach Anspruch 1, wobei der prozentuale Gehalt der Beschichtungsschicht 0,10 bis 1,00 Massen-% beträgt.

3. Kosmetikum, enthaltend die Glasflocken nach Anspruch 1.

## Revendications

1. Flocons de verre chacun consistant en :
un substrat de flocon de verre et
une couche de revêtement revêtant la surface du substrat de flocon de verre, où :
les substrats de flocons de verre présentent une épaisseur moyenne de 0,1 à 1,0 µm, et une taille moyenne de particule de 1 à 100 µm ;
la couche de revêtement est formée de méthylhydrogéno-silicone ; et
le pourcentage de teneur de la couche de revêtement est de 0,05 à 2,50 % en masse comme déterminé en soumettant lesdits flocons de verre à un séchage à 110 ± 5°C pendant au moins 60 minutes suivi par un allumage à 625 ± 20°C pendant au moins 15 minutes, et en déterminant ensuite la réduction de masse des flocons de verre due à cet allumage de sorte que le pourcentage de la perte à partir de la masse des flocons de verre avant l'allumage est calculé.

2. Flocons de verre selon la revendication 1, où ledit pourcentage de teneur de la couche de revêtement est de 0,10 à 1,00 % en masse.

3. Cosmétique contenant les flocons de verre selon la revendication 1.
